(19) Europäisches Patentamt
European Patent Office
Office européen des brevets

(11) EP 0 621 250 B1

# (12) FASCICULE DE BREVET EUROPEEN

(45) Date de publication et mention de la délivrance du brevet:
**05.01.2000 Bulletin 2000/01**

(51) Int Cl.[7]: **C07B 37/02**

(21) Numéro de dépôt: **94400822.6**

(22) Date de dépôt: **14.04.1994**

## (54) Procédé de codimèrisation de diènes et d'oléfines

Verfahren zur Codimerisierung von Dienen und Olefinen

Process for the codimerisation of dienes and olefins

(84) Etats contractants désignés:
**BE DE ES FR GB IT NL**

(30) Priorité: **23.04.1993 FR 9304922**

(43) Date de publication de la demande:
**26.10.1994 Bulletin 1994/43**

(73) Titulaire: **INSTITUT FRANCAIS DU PETROLE**
**92502 Rueil-Malmaison (FR)**

(72) Inventeurs:
- **Stern, Robert**
  **F-75017 Paris (FR)**
- **Kotoko, Abakar**
  **Kousseri (CM)**
- **Hillion, Gérard**
  **F-95220 Herblay (FR)**
- **Chauvin, Yves**
  **F-78230 Le Pecq (FR)**

(56) Documents cités:
**EP-A- 0 475 386**
**WO-A-91/11428**
**DE-A- 4 002 008**
**US-A- 3 502 738**
**US-A- 3 636 122**
**US-A- 3 640 898**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 621 250 B1

## Description

L'invention a pour objet un procédé d'obtention de co-dimères par addition d'une oléfine à des diènes variés, particulièrement des diènes de corps gras.

Il est connu de faire réagir des oléfines avec le butadiène ou autres diènes, avec des complexes du rhodium. De telles réactions sont décrites par exemple dans les documents antérieurs suivants : les brevets US-A-3013066 et US-A-3636122, qui décrivent l'utilisation d'un complexe de rhodium (I) formé avec un ligand cyclopentadiène.

En outre, le brevet US-A-3640898 décrit l'utilisation de complexes au rhodium composés d'un sel de rhodium ou d'un dimère et d'un amide ou d'un oxyde de phosphine amidé.

On connait aussi du brevet US-A-3502738 l'utilisation de complexes dimériques du rhodium dans lesquels le contre-ion est un sel alcalin, l'atome d'hydrogène $H^+$ ou l'ammonium non substitué $NH_4^+$ . Enfin, plus récemment, la demande de brevet internationale WO-A-91/11428 décrit l'utilisation d'une série de complexes au rhodium ou de sels de rhodium pour effectuer l'addition d'éthylène sur des esters linoléiques ou des esters linoléiques qui ont subi une conjugaison de leurs liaisons éthyléniques.

Lorsque le diène utilisé a ses doubles liaisons peu accessibles, la réaction est très lente, comme en témoignent les temps de 24 heures mis en oeuvre pour effectuer la réaction entre l'éthylène et les esters linoléiques dans le document WO-A-91/11428. Il apparait ainsi qu'il y a une difficulté à ajouter de façon économique de l'éthylène à un dérivé diénique de corps gras.

Dans le document EP -A-0 475 386 sont décrits des complexes du rhodium cationique, $[L1RhL2L3R]^+ X^-$, où L1 est un ligand pentahapto-anionique, L2 et L3 sont des ligands neutres donneurs de deux électrons, R est choisi dans le groupe consistant en l'atome d'hydrogène, les radicaux alkyles en C1 à C10, aryles en C6 à C10 et les ligands aralkyles en C7 à C10 et $X^-$ est un anion non coordinant. Ces complexes sont utilisés pour catalyser la préparation d'oléfines linéaires fonctionnalisées, par réaction de deux oléfines terminales, dont l'une au moins porte un radical phényle ou phényle substitué par au moins un groupe alkyle, ou un groupement fonctionnel ester, amide ou aldéhyde.

L'invention propose un procédé permettant d'accélérer l'addition d'oléfine sur des diènes de corps gras notamment sur des diènes conjugués par utilisation d'un système catalytique au rhodium bien spécifique.

Le procédé de l'invention peut être défini d'une manière générale en ce qu'il comprend la réaction d'un composé monooléfinique avec un composé dioléfinique (ou diénique) en présence d'un système catalytique comprenant un composé de rhodium et un sel quaternaire, ce système pouvant être représenté par la formule générale.

$$[RhX_4][YR_4]$$

Dans laquelle X représente un ion halogénure ou un groupement $SO_4^-$, $SO_3^-$, $OH^-$, $OR^-$ ou $R^-$, au moins un X étant un ion halogénure, Y représente un atome d'azote ou un atome de phosphore, et R représente un radical alkyle, cycloalkyle, alkényle, aryle, alkylaryle, polystyryle, alkylpolystyryle ou acyle, les radicaux R pouvant être différents entre eux.

Le composé monooléfinique mis en jeu dans la réaction peut consister en toute monooléfine réactive, choisie parmi les monooléfines ordinaires (hydrocarbure monooléfinique), telles que par exemple l'éthylène, le propylène ou le butène-1, les monooléfines halogénées, telles que par exemple le chlorure de vinyle, et les composés monooléfiniques portant divers groupes fonctionnels, notamment carboxyliques ou nitriles, comme par exemple les acrylates d'alkyle, en particulier l'acrylate de méthyle, l'acrylonitrile, l'acétate d'allyle ou l'acétate de vinyle.

Le composé dit "diénique" mis en jeu dans la réaction sur laquelle est basé le procédé de l'invention est en général un composé comprenant au moins deux liaisons éthyléniques, ces liaisons éthyléniques pouvant être conjuguées ou conjugables deux à deux. Il peut consister en un composé diénique ordinaire (un hydrocarbure diénique) conjugué tel que par exemple le butadiène-1,3, le pipérylène ou l'isoprène, ou en au moins un composé diénique, triénique ou polyénique à fonction carboxylique tel que par exemple les acides gras ayant par exemple de 14 à 26 de préférence de 18 à 24 atomes de carbone, ou leurs esters, notamment ceux dérivant ou contenus dans les huiles naturelles d'origine végétale ou animale, (les huiles de tournesol, de carthame, de poisson, de lin, etc., ou à une concentration moindre, dans les huiles polyinsaturées, comme par exemple les huiles de colza, d'arachide, de noix, etc.).

Il peut encore consister en un composé diénique cyclique (par exemple le cyclopentadiène, le cyclohexadiène, le cyclooctadiène, le cyclododécatriène ou le norbornadiène, ou encore le dicyclopentadiène).

Les acides gras considérés (diéniques, triéniques ou même tétraéniques peuvent être conjugués ou conjugables et utilisés tels quels ou sous la forme des esters qu'ils forment avec des alcools de 1 à 18 atomes de carbone, par exemple des alcools monofonctionnels (tels que le méthanol ou l'éthanol), difonctionnels (tels que le néopentylglycol) ou trifonctionnels (tels que le triméthylol propane ou le glycérol).

Par composés polyoléfiniques conjugables on entend dans l'invention les composés contenant au moins deux

doubles liaisons éthyléniques séparées entre elles par 1 ou 2 atomes de carbone.

Dans le système catalytique utilisé le composé de rhodium est de préférence le chlorure de rhodium hydraté, et le sel quaternaire peut être du type :

$$NR_4X \text{ ou } PR_4X$$

où R et X sont définis comme ci-dessus; de préférence R est un radical méthyle, éthyle, propyle, butyle, pentyle, hexyle, octyle, décyle, dodécyle, hexadécyle, octadécyle, benzyle, polystyryle ou méthylpolystyryle; X représente de préférence un anion halogénure ou sulfate ($SO_4^{--}$), ou l'anion sulfonate ($SO_3^-$) d'une résine, ou encore un anion hydroxyle ($OH^-$).

La mise en oeuvre du catalyseur est réalisée en général au sein d'un solvant choisi par exemple parmi le chloroforme, le dichloroéthane, les alcools (tels que le méthanol ou l'éthanol), les hydrocarbures saturés (tels que l'heptane ou le dodécane) ou aromatiques ou alkylaromatiques (tels que le benzène, le toluène ou un xylène) et les mélanges de ces solvants.

La réaction est effectuée sous une pression allant par exemple de 0,1 à 30 MPa de préférence de 0,4 à 3 MPa, et à une température de 20 à 160 °C, de préférence 80 à 120 °C.

Les produits obtenus par le procédé de l'invention sont des co-dimères ou des mélanges de co-dimères présentant sur la chaine "diénique" 1 ou 2 ramifications, ou même 3 ramifications si l'on utilise par exemple un composé à trois liaisons éthyléniques, comme par exemple l'acide linolénique ou un ester de cet acide.

Les produits obtenus par le procédé de l'invention pour lesquels les composés polyoléfiniques de départ sont des composés à fonction carboxylique, tels que des acides gras ou leurs esters, ont des applications notamment dans la préparation de lubrifiants et d'émulsifiants.

Les exemples suivants illustrent l'invention :

## EXEMPLE 1

Dans cet exemple on a testé les effets de différents sels quaternaires en présence de chlorure de rhodium trihydraté.

L'intérêt des sels quaternaires est mis en évidence. On montre que la sélectivité en produit 1:1 (1 éthylène pour un ester) est excellente et la vitesse décuplée par rapport à un système sans sel quaternaire. Il y a cependant des différences entre les sels quaternaires.

Dans chacun des essais, la solution catalytique est constituée de 0,37 mmol de $RhCl_3, 3H_2O$ et de 0,37 mmol de sel quaternaire dans 15 ml de chloroforme.

176,8 mmol d'ester méthylique de l'huile de tournesol, conjugué à 54 % sont introduits dans 50 ml d'heptane et une pression de 2 MPa est établie à la température de 100 °C. La réaction est effectuée à pression constante.

Le rapport molaire entre l'ester et le chlorure de rhodium est de l'ordre de 480; mais pour l'essai 10, réalisé sans heptane, le rapport est de 780.

TABLEAU 1

| N° | Sel quaternaire | Temps heure | Prd 1:1 % poids | Prd 2:1 % poids |
|---|---|---|---|---|
| 1 | TeMB A | 2 | 4,5 | 0 |
| 2 | TriMSB A | 2 | 20 | 0 |
| 3 | TePB A | 2 | 29,3 | 0 |
| 4 | TeBuB A | 2,75 | 70 | 0 |
| 5 | TeHB A | 2 | 81,6 | 0 |
| 6 | TeHIA | 2 | 2 | 0 |
| 7 | TeOFA | 5 | 40,5 | 0 |
| 8 | BDSCI | 2,3 | 74,4 | 8 |
| 9 | TeBuCI | 2 | 71 | 2 |
| 10 | TePhBrP | 0,6 | 1 | 0 |
| 11 | 0 | 2,5 | 2,5 | 0 |

| | |
|---|---|
| Prd 1:1 | 1 éthylène/1 ester gras, |
| Prd 2:1 | 2 éthylène/1 ester gras, |
| TeMB A | Tétraméthyl bromure d'ammonium, |
| TriMSB A | Triméthyl stéaryl bromure d'ammonium, |
| TePB A | Tétrapropyl bromure d'ammonium, |
| TeBuB A | Tétrabutyl bromure d'ammonium, |
| TeHB A | Tétrahexyl bromure d'ammonium, |
| TeHIA | Tétrahexyl iodure d'ammonium, |
| TeOF A: | Tétraoctyl fluorure d'ammonium. |
| BDSCI A | Benzyl diméthyl stéaryl chlorure d'ammonium mono hydraté, |
| TeBuCIP | Tétrabutyl chlorure de phosphonium, |
| TePhBrP | Tétraphényl bromure de phosphonium, |

Les composés les plus hydrophobes sont en général plus actifs.

Trois caractéristiques du sel quaternaire ont un effet sur la réactivité du chlorure de rhodium. Il s'agit de la longueur du groupe alkyle, la nature de l'halogène et la nature de l'atome présent au centre du sel quaternaire.

L'activité du catalyseur augmente en règle générale avec le nombre d'atomes, de carbone du groupe alkyle. On obtient ainsi un système moins actif avec le tétraméthyl bromure d'ammonium qu'avec le tétrapropyl bromure d'ammonium. Si on remplace un seul groupe méthyle par une chaîne plus longue (Essai n° 2), l'effet négatif du groupe méthyle continue d'influencer le catalyseur. Si on remplace deux groupes méthyles par des groupes plus importants (Essai n° 8), c'est l'effet positif des longues chaînes qui l'emporte. Le remplacement de tous les groupes méthyles par des groupes phényles (Essai n° 10) donne un système quasi inactif, peut-être à cause de l'encombrement.

Plus important pour l'activité du catalyseur semble être la nature de l'halogène du sel quaternaire. Les sels quaternaires à base de chlorure sont les plus actifs (Essais n° 8 et 9); vient ensuite le brome, puis le fluore ; l'iode est pratiquement inactif.

## EXEMPLE 2

Dans cet exemple, on a fait agir deux systèmes catalytiques d'une part à base de $[RhCl_3Br][NBu_4]$ et d'autre part, de $[RhCl_3Br][PBu_4]$, formé chacun à partir d'un mélange stoechiométrique 1:1 de sel quaternaire et de trichlorure de rhodium.

On constate une légère supériorité du sel de phosphonium du point de vue de la conversion, sinon les deux systèmes sont équivalents. Malgré les 4 heures de temps de réaction, la formation du produit 2:1 est minime dans les deux cas et ceci en raison de l'utilisation du bromure.

TABLEAU 2

| COMPARAISON D'UN SEL DE PHOSPHONIUM ET D'UN SEL D'AMMONIUM | | | | |
|---|---|---|---|---|
| **Sel quaternaire** | **$C_{18:2}$ ct % poids** | **$C_{18:2}$ tt % poids** | **Prd 1:1 % poids** | **Prd 2:1 % poids** |
| $NBu_4Br$ | 3,4 | 15 | 78,6 | 2,8 |
| $PBu_4Br$ | 2 | 8,5 | 85,5 | 4,1 |

| Conditions de réaction : | |
|---|---|
| $RhCl_3, 3H_2O$ | 0,74 mmol. |
| Sel quaternaire | 0,74 mmol. |
| Température | 90-100 °C. |
| Pression | 2 MPa. |
| Temps | 4 heures. |

## EXEMPLE 3

Dans cet exemple on utilise un système constitué de chlorure de rhodium et d'un bromure de tétrabutylammonium à la stoechiométrie 1:2 (1 rhodium pour 2 sels quaternaires).

On obtient une sélectivité meilleure qu'à l'exemple 2. Les sels quaternaires à base de bromure peuvent ainsi être utilisés pour une synthèse sélective de produit d'addition 1:1. On constate de plus que la solution est particulièrement homogène même après la réaction.

TABLEAU 3

| Echantillons N° | Temps minutes | Prd 1:1 % poids | Prd 2:1 % poids |
|---|---|---|---|
| 1 | 30 | 35 | 0 |
| 2 | 60 | 56 | 0 |
| 3 | 135 | 79 | 0 |

| | |
|---|---|
| $RhCl_{3,}$ $3H_2O$ | 0,37 mmol |
| $NBu_4Br$ | 0,74 mmol |
| $CHCl_3$ | 25 ml |
| $C_{18:2}$ conjugué | 176 mmol |
| Température | 120 °C |
| Pression | 3 MPa |

La réaction d'addition suit un ordre 1 par rapport au substrat de départ.

## EXEMPLE 4

Dans cet exemple, on a modifié systématiquement le rapport Rh/P. Avec un rapport Rh/P de 1/4, on a constaté une désactivation du catalyseur. Ce dernier système est cependant remarquablement sélectif en produit d'addition 1: 1. Toutefois, la formation de dimères est favorisée dans des réactions de longues durées (4-5 heures). La désactivation du catalyseur augmente avec l'augmentation du sel de phosphonium par rapport au rhodium. Ainsi obtient-on un système presque inactif lorsque le rapport Rh/P est de 1/10. Le sel de phosphonium est le bromure de tétrabutyle phosphonium.

Les résultats sont portés dans le tableau suivant :

## TABLEAU 4

| N° | Rh/P | Temps min | $C_{1:2}$ ct % Poids | $C_{18:2}$ tt % Poids | Prd 1:1 % Poids |
|---|---|---|---|---|---|
| 1 | 1/4 | 156 | 10 | 20 | 70 |
| | | 227 | 7 | 16 | 71* |
| | | 427 | 1 | 12,8 | 76* |
| 2 | 1/6 | 120 | 55 | 28 | 17 |
| 3 | 1/10 | 60 | 77 | 14,5 | 8,5 |
| | | 130 | 73 | 17 | 10 |

ct = cis trans
tt = trans-trans

* : Présence de dimères,
$RhCl_3$, $3H_2O$ : 0,23 mmol (Essai n° 1), 0,38 mmol (Essais n° 2 et 3),
$C_{18:2}$ conjugué : 173 mmol (Essai n° 1), 109 mmol (Essai n° 2) et 164 mmol (Essai n° 3).
Température : 100-120 °C.
Pression : 3 MPa

**EXEMPLE 5**

Dans cet exemple on montre que l'on peut travailler avec très peu de catalyseur.

Le rapport molaire ester méthylique de l'huile de tournesol conjugué/rhodium est de 1917 et le rapport poids ester /Rh est de 5500.

TABLEAU 5

| CODIMERISATION D'ESTER METHYLIQUE DE L'HUILE DE TOURNESOL CONJUGUE ET D'ETHYLENE A FAIBLE CONCENTRATION DE CATALYSEUR | | | | |
|---|---|---|---|---|
| **N°** | **Temps min** | **$C_{18:2}$ ct % Poids** | **$C_{18:2}$ tt % Poids** | **Prd 1:1 % Poids** |
| 1 | 54 | 22 | 39,6 | 38 |
| 2 | 130 | 13,5 | 23 | 63 |
| 3 | 190 | 7,2 | 17 | 77 |

| | |
|---|---|
| $RhCl_3$, $3H_2O$ | 0,14 mmol, |
| PBu4Cl | 0,14 mmol, |
| Température | 100 °C, |
| Pression | 3 MPa, |
| Ester | 268 mmol, |

Ici encore, on constate qu'il faut pratiquement autant de temps pour arriver à 50 % conversion, que pour

passer de 50 à 75 %, ce qui démontre que la réaction est d'ordre 1. Le catalyseur n'est pas détruit. Le temps de 1/2 réaction est de l'ordre de 90 min.

## EXEMPLE 6

Dans cet exemple, on montre la flexibilité d'un système catalytique selon l'invention, particulièrement actif même à des pressions faibles.

Les réactifs sont les suivants : 0,74 mmol de $RhCl_3$, $3H_2O$ ; 0,74 mmol de $PBu_4Cl$; 25 ml de $CHCl_3$ ; 290 mmol de $C_{18:2}$ conjugué.

La pression est de 0,3 à 0,4 MPa et la température de 96 °C.

On obtient principalement du produit 1:1. Après 3 heures de temps de réaction on a 29 % de produit 1:1.

## EXEMPLE 7

Le catalyseur est synthétisé comme dans les exemples précédents, sauf qu'on utilise du THF au lieu de $CHCl_3$.

TABLEAU 6

| INFLUENCE DU THF | | | | |
|---|---|---|---|---|
| **N°** | **Temps min** | **$C_{18:2}$ Ct % Poids** | **$C_{18:2}$ tt % Poids** | **Prd 1:1 % Poids** |
| 1 | 20 | 64 | 22,8 | 13 |
| 2 | 39 | 38 | 27,6 | 30,4 |
| 3 | 80 | 24,5 | 32 | 43 |
| 4 | 146 | 19 | 29,5 | 51,5 |
| 5 | 186 | 16 | 28 | 55,6 |

| | |
|---|---|
| $RhCl_3$, $3H_2O$ | 0,37 mmol, |
| $PBu_4$ Cl | 0,37 mmol, |
| THF | 15 ml, |
| $C_{18:2}$ conjugué | 91 mmol, |
| Température | 95-110 °C, |
| Pression | 2 MPa. |

Le THF est un moins bon solvant que le chloroforme. Néanmoins le système $[RhCl_4][PBu_4]$ dans du THF reste plus performant que le système $RhCl_3$, $3H_2O$ seul dans le chloroforme. On constate ici que le système ne suit pas l'ordre 1. Il y a désactivation progressive du catalyseur.

## EXEMPLE 8

Dans cet exemple, on montre la différence de réactivité entre un chlorure de rhodium et un composé à sel quaternaire. La réactivité du chlorure de rhodium est si faible qu'il a fallu utiliser plus de rhodium que d'habitude pour établir une comparaison.

<u>Essai n° 8.1</u> : Système à sel quaternaire.

TABLEAU 7

| **N°** | **Temps min** | **$C_{18:2}$ ct % Poids** | **$C_{18:2}$ tt % Poids** | **Prd 1:1 % Poids** | **Prd 2:1 % Poids** |
|---|---|---|---|---|---|
| 1 | 49 | 8,4 | 16 | 70 | 3,9 |
| 2 | 104 | 0 | 0 | 66,8 | 31,4 |
| 3 | 184 | 0 | 0 | 51 | 49 |

TABLEAU 7 (suite)

| N° | Temps min | $C_{18:2}$ ct % Poids | $C_{18:2}$ tt % Poids | Prd 1:1 % Poids | Prd 2:1 % Poids |
|---|---|---|---|---|---|
| 4 | 284 | 0 | 0 | 45 | 55 |

| | |
|---|---|
| $RhCl_3$, $3H_2O$ | 1,48 mmol, |
| $PBu_4Cl$ | 1,48 mmol, |
| $CHCl_3$ | 25 ml, |
| Heptane | 100 ml, |
| $C_{18:2}$ conjugué | 91 mmol, |
| Température | 95-100 °C, |
| Pression | 2 MPa. |

Dans l'heptane on constate également une assez grande réactivité isomèrisante puisque, après 50 minutes de réaction, il ne reste plus que 8,4 % de composé Cis-trans.

Essai n° 8.2 : Essai sans sel quaternaire.

TABLEAU 8

| N° | Temps min | $C_{18:2}$ ct % Poids | $C_{18:2}$ tt % Poids | Prd 1:1 % Poids | Prd 2:1 % Poids |
|---|---|---|---|---|---|
| 1 | 50 | 68 | 23,5 | 8,5 | 0 |
| 2 | 164 | 32 | 40 | 26 | 0 |
| 3 | 249 | 21,3 | 37,2 | 38 | 1,6 |
| 4 | 364 | 13 | 31 | 54 | 2 |
| 5 | 639 | 9 | 17 | 69 | 5 |
| 6 | 844 | 3,7 | 13,4 | 71 | 13 |
| 7 | 1200 | 0 | 8 | 73 | 19 |

Les conditions réactionnelles sont les mêmes que pour l'essai n° 8.1.

Pour faire disparaître des diènes, il faut 104 minutes avec le système au sel quaternaire et plus de 1200 minutes avec le chlorure de rhodium; en fait, si l'on considère un ordre 1 par rapport au substrat, la réaction du tableau 7. va 15 fois plus vite et fait disparaître complètement le diène.

**EXEMPLE 9**

Dans cet exemple, on montre que certains phosphoranes donnent des résultats semblables à ceux du sel quaternaire de phosphonium.

TABLEAU 9

| SYSTEME AU PHOSPHORANE | | | | | |
|---|---|---|---|---|---|
| N° | Temps min | $C_{18:2}$ ct % Poids | $C_{18:}2$ tt % Poids | Prd 1:1 % Poids | Prd 2:1 % Poids |
| 1 | 96 | 0 | 2 | 89 | 9 |
| 2 | 181 | 0 | 0 | 83 | 17 |

| | |
|---|---|
| $RhCl_{3,}$ $3H_2O$ | 0,37 mmol, |
| Phosphorane | 0,37 mmol, |
| Chloroforme | 20 ml, |

(suite)

| | |
|---|---|
| $C_{18:2}$ conjugué | 171 mmol, |
| Température | 140 °C, |
| Pression | 2,5 MPa |

Le phosphorane a la formule

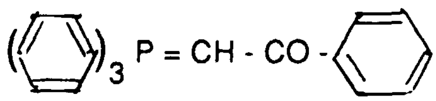

Pour former 98 % de produit converti, il faut 96 minutes ce qui correspond à 310 moles de produit formé/heure/mole de rhodium en admettant un ordre pour le catalyseur, ce qui n'est pas exactement le cas. Avec le chlorure de rhodium il faut selon l'exemple précédent 1200 minutes pour obtenir une conversion totale, ce qui correspond à 3 moles de produit formé/mole de rhodium/heure. Le rapport en activité est de l'ordre de 100.

## EXEMPLE 10

Dans cet exemple on utilise un catalyseur sur support.

On agite dans un ballon 0,1 gramme (0,37 mmol) de $RhCl_3$, $3H_2O$ et 1 gramme (0,85 mmol) de tributyl polystyrène chlorure d'ammonium (produit vendu par la Société Flucka) dans 20 ml de chloroforme. On laisse ainsi agir pendant 5 heures. La résine fixant le rhodium est filtrée et lavée au chloroforme puis séchée. Le filtrat est incolore.

149 mmol d'ester méthylique d'huile de tournesol conjugué à 52 % et 60 ml de n-heptane sont utilisés. On charge 2 MPa d'éthylène et on chauffe à 110 °C. Le rapport sel quaternaire/Rh est ici de 2,29.

TABLEAU 10

| N° | Temps min | $C_{18:2}$ tc % Poids | $C_{18:2}$ tt % Poids | Prd 1:1 % Poids |
|---|---|---|---|---|
| 1 | 30 | 59 | 21,3 | 19 |
| 2 | 191 | 24 | 25,8 | 33 |
| 3 | 371 | 11,5 | 20,7 | 64,4 |

On constate dans cet essai, qu'il y a un ralentissement net après 30 minutes de réaction. La vitesse décroit d'un facteur 3.

## EXEMPLE 11

On synthétise la solution catalytique à partir de 0,74 mmol de trichlorure de rhodium tri hydraté et de 1,48 mmol de tétrabutyl chlorure de phosphonium dans 10 ml d'eau. On charge 299 mmol d'ester méthylique de l'huile de tournesol conjugué à 52 % et 2 MPa d'éthylène à une température de 80 °C. On laisse réagir pendant 195 minutes et on obtient 3,4 % poids de produit d'addition 1:1. Le système biphasique à l'eau ne semble pas dans ce cas très actif.

## EXEMPLE 12

Dans cet exemple, on montre que le catalyseur est particulièrement actif même avec des esters non conjugués, soit le linoléate de méthyle.

TABLEAU 12

| REACTION DE L'ESTER METHYLIQUE DE L'HUILE DE TOURNESOL AVEC L'ETHYLENE | | | | | |
|---|---|---|---|---|---|
| N° | Temps min | $C_{18:2}$ % Poids | $C_{18:2}$ ct % Poids | $C_{18:2}$ tt % Poids | Prd 1:1 % Poids |
| 1 | 0 | 100 | 0 | 0 | 0 |
| 2 | 14 | 65 | 10 | 13 | 12 |

TABLEAU 12 (suite)

| REACTION DE L'ESTER METHYLIQUE DE L'HUILE DE TOURNESOL AVEC L'ETHYLENE | | | | | |
|---|---|---|---|---|---|
| **N°** | **Temps min** | **$C_{18:2}$ % Poids** | **$C_{18:2}$ ct % Poids** | **$C_{18:2}$ tt % Poids** | **Prd 1:1 % Poids** |
| 3 | 43 | 59,5 | 7,5 | 12 | 21 |
| 4 | 103 | 54,8 | 4,4 | 9,6 | 33,4 |
| 5 | 163 | 44,5 | 3 | 7,5 | 44,9 |
| 6 | 278 | 38,7 | 1,5 | 6,8 | 50,1 |

| | |
|---|---|
| $RhCl_3,3H_2O$ | 0,44 mmol, |
| $PBu_4Cl$ | 0,44 mmol, |
| C18:2 | 161,5 mmol |
| Température | 100 °C, |
| Pression | 3 MPa. |

Les chiffres donnés au tableau 12 correspondent au diène présent dans l'ester méthylique de tournesol.

Il ressort du tableau 12 que l'ester linoléique est d'abord conjugué en cis/trans et ensuite en isomère trans/trans avant d'être additionné d'éthylène. La conjugaison est prédominante dans la première partie de la réaction. En fin de réaction, on constate le changement de couleur de la solution réactionnelle. Elle passe du rouge au noir, alors qu'avec un ester conjugué elle reste rouge. On peut dire que l'étape de la conjugaison est l'étape lente de la réaction et que le catalyseur se désactive au fur et à mesure de la réaction comme si la présence de diène conjugué au départ stabilisait le catalyseur.

## EXEMPLE 13

Dans cet exemple, on montre la réactivité de ce système catalytique sur de simples diènes conjugués. On fait ainsi réagir le pipérylène avec l'éthylène.

La solution catalytique est constituée de 0,74 mmol de trichlorure de rhodium tri hydraté, 0,74 mmol de tétrabutyl chlorure de phosphonium de 30 ml de chloroforme. On charge 501 mmol de pipérylène et 2 MPa d'éthylène. On laisse réagir pendant 60 minutes à 80 °C et on obtient 96 % de 3-méthyl 1,4 hexadiène, 3 % de pipérylène et des traces de produit plus lourd.

Par distillation, sous argon, on sépare le produit obtenu du catalyseur. Le reste de la distillation est ainsi réutilisé comme solution catalytique dans une nouvelle réaction d'addition. Dans les mêmes conditions que la réaction précédente on fait réagir 501 mmol de pipérylène avec de l'éthylène pendant 215 minutes. On obtient 42 % de 3-méthyl 1,4 hexadiène pour une conversion de 42 %.

## EXEMPLE 14

Dans cet exemple on fait agir 299 mmol d'acide de l'huile de tournesol conjugué à 57 % avec de l'éthylène en présence d'une solution catalytique composée de 1,1 mmol de trichlorure de rhodium et de 1,1 mmol de tétrabutyl chlorure de phosphonium dans 25 ml de chloroforme. La réaction est faite à 90 °C et sous 2MPa d'éthylène. On obtient après 7h30 de temps de réaction le mélange de produit suivant après hydrogénation.

72 % poids de produit 1:1, 19 % poids de produit 2:1 (diéthyl) et 5 % poids de produit 2:1 (isobutyl).

## Revendications

1. Procédé d'obtention d'un co-dimère caractérisé en ce que l'on additionne au moins un composé monooléfinique sur un composé comportant au moins 2 liaisons éthyléniques, conjuguées ou non conjuguées, en présence d'un système catalytique comprenant un composé du rhodium et un sel quaternaire et répondant à la formule générale.

$$[Rh\ X_4]\ [YR_4]$$

dans laquelle X représente un ion halogénure ou un groupement $SO_4^{--}$, $SO_3^-$, $OH^-$, $OR^-$ ou $R^-$, au moins un des X étant un halogénure, Y représente un atome d'azote ou un atome de phosphore et R représente un radical alkyle, cycloalkyle, alkényle, aryle, alkylaryle, polystyryle, alkylpolystyryle ou acyle.

**2.** Procédé selon la revendication 1, caractérisé en ce que ledit composé monoléfinique est choisi parmi les hydrocarbures monooléfiniques, les monooléfines halogénées et les composés monooléfiniques portant des groupements carboxyliques ou nitriles.

**3.** Procédé selon la revendication 2, caractérisé en ce que ledit composé monooléfinique est choisi parmi l'éthylène, le propylène, le butène-1, le chlorure de vinyle, l'acrylate de méthyle, l'acrylonitrile, l'acétate d'allyle et l'acétate de vinyle.

**4.** Procédé selon l'une des revendications 1 à 3, caractérisé en ce que ledit composé comportant au moins deux liaisons éthyléniques est choisi parmi les diènes conjugués ou conjugables, aliphatiques ou cycliques, et les composés diéniques, triéniques ou tétraéniques à fonctions carboxyliques dont les liaisons éthyléniques sont conjuguées ou conjugables.

**5.** Procédé selon la revendication 4, caractérisé en ce que ledit composé comportant au moins deux liaisons éthyléniques est choisi parmi le butadiène -1,3, le pipérylène et l'isoprène.

**6.** Procédé selon la revendication 4, caractérisé en ce que ledit composé comportant au moins deux liaisons éthyléniques est choisi parmi les acides monocarboxyliques de 14 à 26 atomes de carbone et les esters qu'ils forment avec des alcools aliphatiques mono-, di-, ou trifonctionnels, de 1 à 18 atomes de carbone.

**7.** Procédé selon la revendication 6, caractérisé en ce que ledit alcool est choisi parmi le méthanol, l'éthanol, le néopentylglycol, le triméthylolpropane et le glycérol.

**8.** Procédé selon l'une des revendications 6 et 7, caractérisé en ce que lesdits acides monocarboxyliques et lesdits esters dérivent d'huiles naturelles d'origine végétale ou animale.

**9.** Procédé selon l'une des revendications 1 à 8, caractérisé en ce que le système catalytique comprend, à titre de composé du rhodium, du chlorure de rhodium hydraté et, à titre de sel quaternaire, un composé du type $NR_4X$ ou $PR_4X$, où R est un radical méthyle, éthyle, propyle, butyle, pentyle, hexyle, octyle, décyle, dodécyle, hexadécyle, octadécyle, benzyle, polystyryle ou méthylpolystyryle.

**10.** Procédé selon l'une des revendications 1 à 9 caractérisé en ce que la réaction est effectuée au sein d'au moins un solvant, sous une pression de 0,1 à 30 MPa et à une température de 20 à 160 °C.

## Claims

**1.** A process for the production of a co-dimer, characterised in that at least one monoolefin is added to a compound containing at least two conjugated or unconjugated ethylenic bonds, in the presence of a catalytic system comprising a rhodium compound and a quaternary salt corresponding to the general formula:

$$[RhX_4][YR_4]$$

where X represents a halogen ion or a $SO_4^{--}$ $SO_3^-$, $OH^-$, $OR^-$ or $R^-$ group, at least one X being a halide, Y represents a nitrogen atom or a phosphorous atom and R represents an alkyl, cycloalkyl, alkenyl, aryl, alkylaryl, polystyryl, alkylpolystyryl or acyl radical.

**2.** Process according to claim 1, characterised in that said monoolefin is selected from monoolefinic hydrocarbons, halogenated monoolefins and monoolefinic compounds containing carboxylic or nitrile groups.

**3.** Process according to claim 2, characterised in that said monoolefinic compound is selected from ethylene, propylene, 1-butene, vinyl chloride, methyl acrylate, acrylonitrile, allyl acetate and vinyl acetate.

4. Process according to any one of claims 1 to 3, characterised in that said compound comprising at least two ethylenic bonds is selected from conjugated or conjugatable, aliphatic or cyclic dienes and dienic, trienic or tetraenic compounds containing carboxylic functions wherein the ethylenic bonds are conjugated or conjugatable.

5. Process according to claim 4, characterised in that said compound containing at least two ethylenic bonds is selected from 1,3-butadiene, piperylene and isoprene.

6. Process according to claim 4, characterised in that said compound containing at least two ethylenic bonds is selected from monocarboxylic acids containing 14 to 26 carbon atoms and their esters formed with mono-, di- or trifunctional aliphatic alcohols containing 1 to 18 carbon atoms.

7. Process according to claim 6, characterised in that said alcohol is selected from methanol, ethanol, neopentylglycol, trimethylolpropane and glycerol.

8. Process according to claim 6 or claim 7, characterised in that said monocarboxylic acids and said esters are derived from natural plant or vegetable oils.

9. Process according to any one of claims 1 to 8, characterised in that the rhodium compound in the catalytic system comprises a hydrated rhodium chloride and the quaternary salt comprises a $NR_4X$ or $PR_4X$ type compound, where R is a methyl, ethyl, propyl, butyl, pentyl, hexyl, octyl, decyl, dodecyl, hexadecyl, octadecyl, benzyl, polystyryl or methylpolystyryl radical.

10. Process according to any one of claims 1 to 9, characterised in that the reaction is carried out in at least one solvent at a pressure of 0.1 to 30 MPa and a temperature of 20°C to 160°C.

**Patentansprüche**

1. Verfahren zur Herstellung eines Codimers, dadurch gekennzeichnet, daß man mindestens eine Monoolefin-Verbindung an eine Verbindung, die mindestens zwei konjugierte oder nicht-konjugierte Ethylenbindungen aufweist, in Gegenwart eines katalytischen Systems addiert, das eine Rhodium-Verbindung und ein quaternäres Salz umfaßt und die folgende allgemeine Formel hat:

$$[Rh\ X_4][YR_4]$$

worin bedeuten:

x ein Halogenidion oder eine Gruppe $SO_4^{2-}$, $SO_3^-$, $OH^-$, $OR^-$ oder $R^-$, wobei mindestens einer der Reste X ein Halogenid ist,
Y ein Stickstoff- oder Phosphoratom und
R einen Alkyl-, Cycloalkyl, Alkenyl-, Aryl-, Alkylaryl-, Polystyryl-, Alkylpolystyryl- oder Acyl-Rest.

2. Verfahren nach Anspruch 1, dadurch gekennzeichnet, daß die genannte Monoolefin-Verbindung ausgewählt wird aus den Monoolefin-Kohlenwasserstoffen, den halogenierten Monoolefinen und den Monoolefin-Verbindungen, die Carboxyl- oder Nitril-Gruppen tragen.

3. Verfahren nach Anspruch 2, dadurch gekennzeichnet, daß die genannte Monoolefin-Verbindung ausgewählt wird aus Ethylen, Propylen, Buten-1, Vinylchlorid, Methylacrylat, Acrylnitril, Allylacetat und Vinylacetat.

4. Verfahren nach einem der Ansprüche 1 bis 3, dadurch gekennzeichnet, daß die genannte Verbindung, die mindestens zwei Ethylenbindungen aufweist, ausgewählt wird aus den konjugierten oder konjugierbaren aliphatischen oder cyclischen Dienen und den Dien-, Trien- oder Tetraen-Verbindungen mit Carboxyl-Funktionen, deren Ethylenbindungen konjugiert oder konjugierbar sind.

5. Verfahren nach Anspruch 4, dadurch gekennzeichnet, daß die genannte Verbindung, die mindestens zwei Ethylenbindungen aufweist, ausgewählt wird aus Butadien-1,3, Piperylen und Isopren.

**6.** Verfahren nach Anspruch 4, dadurch gekennzeichnet, daß die genannte Verbindung, die mindestens zwei Ethylenbindungen aufweist, ausgewählt wird aus den Monocarbonsäuren mit 14 bis 26 Kohlenstoffatomen und den Estern, die sie mit mono-, di- oder trifunktionellen aliphatischen Alkoholen mit 1 bis 18 Kohlenstoffatomen bilden.

**7.** Verfahren nach Anspruch 6, dadurch gekennzeichnet, daß der genannte Alkohol ausgewählt wird aus Methanol, Ethanol, Neopentylglycol, Trimethylolpropan und Glycerin.

**8.** Verfahren nach einem der Ansprüche 6 und 7, dadurch gekennzeichnet, daß die genannten Monocarbonsäuren und die genannten Ester aus natürlichen Ölen pflanzlichen oder tierischen Ursprungs stammen.

**9.** Verfahren nach einem der Ansprüche 1 bis 8, dadurch gekennzeichnet, daß das katalytische System als Rhodium-Verbindung hydratisiertes Rhodiumchlorid und als quaternäres Salz eine Verbindung vom Typ $NR_4X$ oder $PR_4X$ umfaßt, worin R für einen Methyl-, Ethyl, Propyl-, Butyl-, Pentyl-, Hexyl-, Octyl-, Decyl-, Dodecyl-, Hexadecyl-, Octadecyl-, Benzyl-, Polystyryl- oder Methylpolystyryl-Rest steht.

**10.** Verfahren nach einem der Ansprüche 1 bis 9, dadurch gekennzeichnet, daß die Reaktion in mindestens einem Lösungsmittel unter einem Druck von 0,1 bis 30 MPa und bei einer Temperatur von 20 bis 160°C durchgeführt wird.